# EUROPEAN PATENT SPECIFICATION

(11) **EP 1 157 680 B1**
(45) Date of publication and mention of the grant of the patent: **28.07.2004**
(21) Application number: 01830052.5
(22) Date of filing: 26.01.2001
(51) Int. Cl.: A61F 13/15, B65D 75/00

(54) **A package for sanitary articles and corresponding manufacturing method**
Ein Paket für Hygieneartikel und Verfahren zur dessen Herstellung
Conditionnement pour articles sanitaires et la méthode de fabrication correspondante

(30) Priority: 23.05.2000 IT TO000465
(43) Date of publication of application: 28.11.2001
(73) Proprietor: Fameccanica.Data S.p.A., 66020 Sambuceto di S. Giovanni Teatino (Chieti) (IT)
(72) Inventor: Pelagatti, Pietro, 66020 Sambuceto di San Giovanni Teatino (IT)
(74) Representative: Bosotti, Luciano

(56) References cited:
- PATENT ABSTRACTS OF JAPAN vol. 1997, no. 01, 31 January 1997 (1997-01-31) & JP 08 224269 A (KAO CORP), 3 September 1996 (1996-09-03)
- PATENT ABSTRACTS OF JAPAN vol. 1997, no. 12, 25 December 1997 (1997-12-25) & JP 09 215707 A (TOA KIKO KK), 19 August 1997 (1997-08-19)
- PATENT ABSTRACTS OF JAPAN vol. 015, no. 416 (M-1172), 23 October 1991 (1991-10-23) & JP 03 176376 A (DAINIPPON PRINTING CO LTD), 31 July 1991 (1991-07-31)

## Description

The present invention relates to packs for sanitary articles, such as panty-liners and similar products.

More specifically, the present invention relates to a pack comprising an article applied on a film or sheet element, the assembly formed by the article and by the film or sheet element, comprising a central branch, as well as a first end branch and a second end branch, and being foldable according to a general C conformation, with said first end branch folded against said central branch and said second end branch folded against said first branch, which is in turn folded against said central branch.

In certain packs of the type specified above currently available on the market, the article contained in the pack is fixed to the latter with the application of adhesive material (see e.g. JP-A-09 215 707).

The adoption of this solution may, however, give rise to a problem linked to the fact that, once the new and clean article is taken out of the pack, the film or sheet element should be advantageously re-usable to envelop (so as to enable disposal thereof) the used article which the user has removed from her garment.

At least in certain environmental conditions and conditions of use, once the article has been taken out of the pack, there remains residue of adhesive on the film or sheet element such as to render the latter as a whole sticky, with a marked tendency towards the formation of folds or pleats the flaps of which, being adhesively connected together, can practically no longer be separated. It is clear that such a film or sheet element can in no way be re-used for the purpose referred to previously.

Some solutions envisage recourse to adhesive materials according to modalities such as to cause the adhesive to leave traces that are scarcely perceptible when the product is separated from the film or sheet element of the pack. These solutions are based, however, on the use of adhesive materials and/or of films of plastic material having particular characteristics and as a result usually quite a high cost.

In general, recourse to adhesive connection means that the operation of detaching the article from the pack may be accompanied by a certain amount of noise that is liable to be perceived at least by some users - as a negative aspect considering the desirability of a discreet use of the article.

The known solutions described previously may then present the drawback that the end of the article associated to the branch of the film or sheet element located in the outermost position when the pack is closed is not sufficiently protected against the possible penetration of agents of contamination from the external environment. In certain solutions, the aforesaid flap is then withheld against the body of the pack by the application of a further adhesive label. All this means that yet another element of the pack must be provided and applied.

The object of the present invention is to overcome the drawbacks of the solutions described previously, at the same time maintaining the advantages of the said solutions.

According to the present invention, the above purpose is achieved thanks to a pack having the characteristics called for specifically in the claims which follow.

The invention also relates to the corresponding manufacturing process.

The present invention will now be described in detail with reference to the attached drawings, which are provided purely by way of non-limiting examples and in which:
- Figure 1 is a general perspective view of part of a production system that may be used to make packs according to the present invention.
- Figure 2 is a view illustrating at an enlarged scale and in greater detail the part of Figure 1 indicated by the arrow II, and
- Figures 3 and 4 illustrate as a whole the characteristics of the pack according to the present invention in two successive steps of the corresponding fabrication process and/or process of use.

The figures of the attached drawings illustrate the possible application of the solution according to the invention to the making of packs P containing articles A consisting, in the example of embodiment illustrated, of sanitary articles, such as panty-liners.

The solution according to the invention may, however, be used for making sanitary articles of different types (e.g., absorbent articles of various nature, including medical articles), it remaining understood that the aforesaid articles need to be foldable according to a general C conformation, i.e., with the formation of a central branch and two end branches, the first folded against the central branch and the second folded against the first end branch.

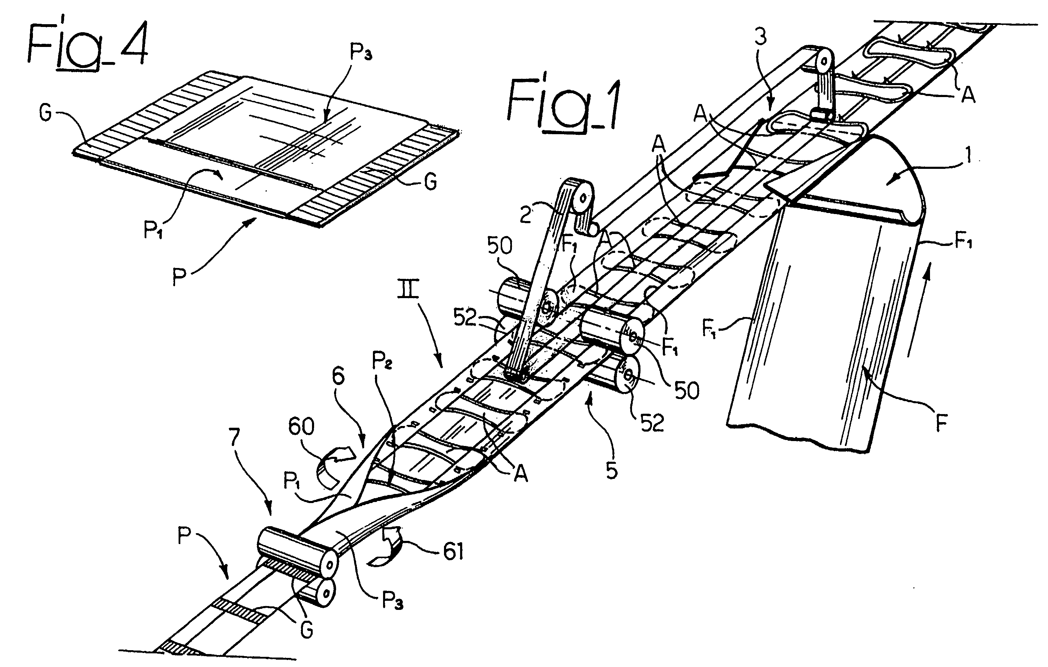

As viewed in Figure 1, the articles A are supposed as being fed (with known means that are not illustrated) in a continuous flow comprising articles A which move forward set sideways on (i.e., with their direction of greater extension orthogonal to the direction of feed) and with a given pitch between each one (for example, in the region of 7-14 cm). As viewed in Figure 1, the said forward movement takes place from right to left and top to bottom.

At a point corresponding to a fitting station, designated as a whole by 1, there is made to converge towards the flow of articles A a web of sheet material F consisting typically of a strip of plastic material, such as polyethylene film.

At a point corresponding to the position of convergence with the articles A, the film F is fed forwards (by motor-powered means, which are not illustrated but of a known type, e.g., motor-driven rollers) so as to advance at the same speed as that of the flow of articles A.

Means for drawing along (consisting, for example, of a motor-driven conveyor belt 2 of a known type) act on the articles A from the opposite side to the one on which the film F is set. Once the articles A have been fitted to the film F, they thus advance with complete synchronism with the film F, hence in conditions of absence of relative movement with respect to the film F, all this being achieved without the need to resort, in order to obtain the desired result, to positive forms of connection involving the application of adhesive material between the articles A and the film F.

In such conditions, the film F and the articles A are fed towards a forming device 3, which is also of a type as a whole known: forming devices of this type are currently present, for instance, in the packaging machines of the type currently referred to as flow-pack in order to enclose the packaging film in the form of a cylinder. In the forming device 3 the longitudinal margins F1 of the film F are turned up and folded against the central portion of the film F, in such a way that the homologous ends of the articles A are entrapped and withheld against the central portion of the film F by the aforesaid turned-up longitudinal margins F1.

The composite ribbon-like element thus obtained, consisting of the film F with the margins F1 turned up against the central portion and of the articles A set thereon with the ends withheld by the aforesaid longitudinal margins, is fed towards a sealing assembly 5. This takes place in such a way that the conveyor means 2 continue to act on the articles A in an area corresponding to the central portion of the latter.

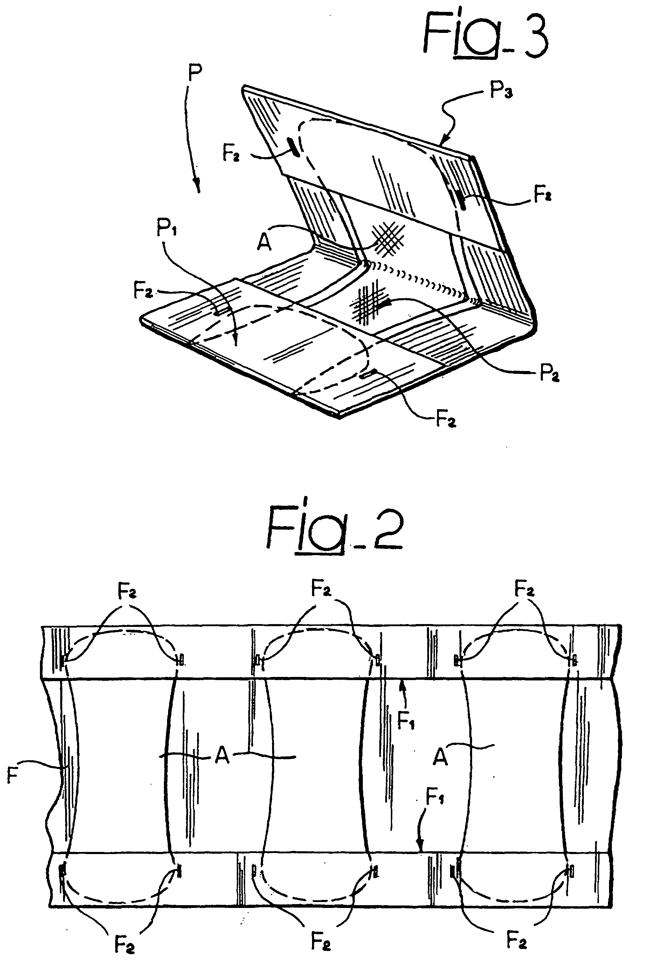

The function of the sealing assembly 5 (also of a generally known type; for instance, it may be a set of counter-rotating jaws, basically resembling the ones used for forming the end sealing lines of the flow-pack packaging machines referred to previously) is that of forming, on both sides of each of the end parts of the articles A, two connection areas; for instance, these may be two sealing lines F2 (see, in particular, Figure 2). The lines F2 are designed to constitute a stable connection of the margins F1 to the central part of the film F against which the aforesaid margins F1 have previously been folded in the forming device 3.

The result that may be obtained from the action of connection described is that each article A presents itself with its two ends inserted and withheld in corresponding pocket parts, with each pocket part defined by:
- a respective margin portion F1 comprised between two successive connection lines F2, and
- the homologous portion of the central part of the film F on which the said margin has previously been folded.

The articles A may therefore be released from the conveying means 2, which do not usually extend beyond the sealing station 5. From this point on, the articles A are in fact firmly withheld on the film F and can thus move together with the film F itself, which is fed along by known motor-driven means, not specifically illustrated in the drawings.

The foregoing is obtained without any need for the application of adhesive material.

The perspective view presented in Figure 1 provides a better illustration of the fact that the sealing station 5 preferably comprises, on either side of the path of advance of the film F, a pair of counter-rotating sealing jaws driven by motor means (not specifically illustrated, but of a known type) which operate in a synchronized way with the movement of feeding of the articles A and of the film F.

Again from Figure 1 it will further be noted that at least two jaws 50 designed to operate on the top face of the film F are installed according to a general cantilever arrangement, hence without the presence of elements of interconnection, such as the shaft (not visible in the figure) which instead connects the homologous jaws, designated by 52, designed to act on the bottom face of the film F.

The fact that the jaws 50 are installed in cantilever fashion thus enables the drawing means 2 to extend as far as the area in which the articles A are firmly received within the pocket formations referred to previously.

The solution described presents the advantage of ensuring exact positioning of the articles in the region where the sealing station 5 acts, preventing the jaws of the sealing station from accidentally acting in an undesired way on the ends of the articles A, an eventuality that could accidentally occur in the absence of a positive guiding action (or more precisely an accompanying action) on the articles A of the type exerted by the means 2.

Downstream of the station 5, the pack-forming process proceeds with operations that basically resemble those adopted in the known art for making the known packs to which reference was made in the introductory part of the present description.

In practice, the composite ribbon or web formed by the film F and by the articles A (having their ends inserted in the pocket formations created in the film F itself) is sent on to a further forming assembly 6 in which an end branch P1 of said composite ribbon is folded against the central branch P2 of the ribbon and, subsequently, the other end branch P3 is folded against the branch P1 so as to complete closing of the pack according to a general C conformation. For simplicity of illustration and in view of its notoriety (it is the same device used for bending into a C shape the packs of a known type to which reference was made in the introductory part of the present description) the presence of the forming assembly 6 has been recalled here in an altogether schematic way, where the successive steps of intervention of the device are represented by the arrows 60 and 61.

In a further sealing and cutting station, designated by 7, which also normally comprises one or more pairs of counter-rotating jaws, the film folded in a C configuration undergoes an operation of sealing and cutting in areas between successive articles A, the aim being to obtain individual packs corresponding to the pack P represented in Figure 3 (in a half-opened condition) and in Figure 4 (in a closed condition).

The pack P thus comprises the article A set with its ends inserted in the pockets formed, according to the criteria described previously, by the margins F1 folded against the central part of the film, the aforesaid pockets being closed at the sides by the connection lines F2.

The assembly thus obtained is folded according to a general C configuration with:
- a central branch P2 consisting of the median part of the film F and of the central part of the article A;
- a first end branch P1, comprising a first end of the article A received in a corresponding pocket, folded against the central branch P2; and
- a second end branch P3, comprising the other end of the article A received in a corresponding pocket, folded against the end branch P1.

The above arrangement is obtained with the aforesaid pack withheld in a closed condition as a result of the connection (typically obtained by heat sealing of the film F in the station 7) of the edges G of the aforesaid portions P1, P2 and P3 extending in the direction of greater extension of the article A.

In use, the user takes out the closed pack (Figure 4), raises the branch P3 away from the branch P1 (see Figure 3), and then raises the branch P1 thus uncovered from the branch P2, in so doing gradually separating the parts of the film element F connected along the edges G.

In this way the article A is directly accessible so that it can be taken out of the pack. The article A may in fact be easily taken out of the pack, for instance by inserting a finger between the central portion of the article A and the homologous portion of the film element of the pack made up of the film F and then sliding the ends of the article A out of the corresponding receiving pockets.

This result is achieved avoiding adhesive connection of the articles A to the film F, even though, of course, the article A may have on one of its sides an adhesive coating designed to enable a firmer connection of the article A to the garment on which the article A is to be worn. The aforesaid adhesive coating is in turn protected by a selectively removable strip, for example a strip of siliconized paper.

It will be appreciated that the above coating and the corresponding strip or protection label do not necessarily have to be applied on the side of the article A designed to be set facing the film F.

The solution according to the invention in fact makes it possible to set the article A in such a way that the aforesaid adhesive coating and the corresponding strip are located on the side of the article A opposite to the film F.

The above location of the adhesive coating and corresponding strip may prove advantageous for at least two reasons, as described below.

In the first place it enables a faster access to the strip (which is to be removed) when the pack is opened and the article taken out.

In the second place, with this arrangement, the side of the article A opposite to the strip (i.e., the side which, in use, is to face the body of the user) remains applied against the film F up to the moment when the article A is taken out of the pack, with the result that it is better protected.

For making the film F it is possible to use any plastic strip-like material which can be manipulated (i.e., shaped, sealed, etc.) in the way described, without it having to present characteristics and/or undergo processes such as to enable easy detachment of the article A, possibly preventing the formation of residue of adhesive.

The article A can be removed from the pack just by sliding it out, hence avoiding the generation of any noise of tearing or detachment, and the film part of the pack is directly re-usable for receiving a used article, which is to be disposed of.

Furthermore, it will be appreciated that in the closed pack P the end of the article A corresponding to the branch P3 is in any case inserted in the corresponding pocket formed by the film F and is thus protected against the possible penetration of contaminating agents from outside, all this without requiring the additional connection of the distal margin of the branch P3 to the first branch P1 (for instance, with the application of an adhesive layer).

Of course, without prejudice to the principle of the invention, the details of construction and the embodiments may vary widely with respect to what is described and illustrated herein, without thereby departing from the scope of the present invention. This applies in particular, but without limitation, to the modalities for making the connection formations represented in the example illustrated by the lines F2. These lines may be produced by sealing (e.g., heat sealing), gluing or equivalent techniques, in a continuous or discontinuous way, with a more or less marked extension according to the specific applicational requirements. In particular, each pair of lines F2 located in a position immediately adjacent to one another without interposition of an article A (hence two lines F2 designed to define distinct corresponding pockets for receiving two successive articles A arriving one after the other in the flow) may be formed starting from a single area of sealing which is subsequently divided into two parts at the moment of separation of two successive packs.

## Claims

1. A pack comprising an article (A) applied on a sheet or film element (F) , the assembly formed by the article (A) and by the film element (F) comprising a central branch (P2) as well as a first end branch (P1) and a second end branch (P3), and being foldable according to a general C conformation, with said first end branch (P1) folded against said central branch (P2) and said second end branch (P3) folded against said first branch (P1), which is in turn folded against said central branch (P2), **characterized in that** said film element (F) comprises, in areas corresponding to said end branches (P1, P3), pocket formations in which the homologous ends of said article (A) are received.

2. The pack according to Claim 1, **characterized in that** said article (A) is a sanitary product such as a panty-liner.

3. The pack according to Claim 1 or Claim 2, **characterized in that** said article (A) is withheld on said film element (F) as a result of the location of said homologous ends in said pocket formations, in the substantial absence of adhesive connection between said article (A) and said film element (F).

4. The pack according to any one of the preceding claims, **characterized in that** said article (A) carries applied to it a protective strip, said protective strip being selectively removable from said article (A).

5. The pack according to Claim 4, **characterized in that** between said article (A) and said protective strip there is interposed an adhesive layer.

6. The pack according to any one of the preceding claims, **characterized in that** said pocket formations are made up of margins (F1) of said film element (F) which are folded against the film element (F) itself.

7. The pack according to Claim 6, **characterized in that** said margins (F1) are connected to said film element by means of connection formations (F2), preferably in the form of lines.

8. The pack according to any one of the preceding claims, **characterized in that** with said pack folded according to said C conformation, said second end branch (P3) is connected to said first end branch (P) P1) in the substantial absence of connection elements between the distal margin of said second end branch (P3) and said first end branch (P1).

9. A process for making a pack (P) comprising an article (A) applied on a strip element (F), the assembly formed by the article (A) and by the film element (F) comprising a central branch (P2) as well as a first end branch (P1) and a second end branch (P3) and being foldable according to a general C conformation, with said first end branch (P1) folded against said central branch (P2) and said second end branch (P3) folded against said first branch (P1), which is folded against said central branch (P2), **characterized in that** it comprises the operation of making in said film element (F), in areas corresponding to said end branches (P1, P3), pocket formations in which the homologous ends of said article (A) are received.

10. The process according to Claim 9, **characterized in that** said article (A) is chosen as a sanitary product such as a panty-liner.

11. The process according to Claim 9 or Claim 10, **characterized in that** it comprises the operation of placing said corresponding homologous ends in said pocket formations, in the substantial absence of adhesive connection between said article (A) and said film element (F).

12. The process according to any one of the preceding claims from Claim 9 to Claim 11, **characterized in that** it comprises the operation of applying to said article (A) a protective strip, said protective strip being selectively removable from said article (A).

13. The process according to Claim 12, **characterized in that** it comprises the operation of interposing an adhesive connection layer between said article (A) and said protective strip.

14. The process according to any one of the preceding claims from Claim 9 to Claim 13, **characterized in that** it comprises the operation of forming said pocket formations by folding (3) margins (F1) of said film element (F) against the film element (F) itself.

15. The process according to Claim 14, **characterized in that** it comprises the operation of connecting said margins (F1) to the body of said film element (F1), preferably by sealing.

16. The process according to any one of the preceding claims from Claim 9 to Claim 15, **characterized in that** it is carried out on a flow of said articles (A) as they are moving and comprises the operation of positively controlling (2) the movement of said articles (A) until said pocket formations are made.

17. A process according to Claim 16, **characterized in that** said pocket formations are formed by acting on said opposed margins (F1) of said film element (F) with connecting elements (5) mounted, at least in part (50), according to a general cantilever configuration with respect to said first film element (F), and **in that** it comprises the operation of providing means (3) for positive conveying of said articles (A) extending in a position between said connecting elements (5) arranged at least in part (50) in cantilever fashion.

## Patentansprüche

1. Ein Packen mit einem Artikel (A), welcher auf einem Blatt- oder einem Folienelement (F) angebracht ist, wobei die Anordnung, die durch den Artikel (A) und das Folienelement (F) gebildet wird, einen zentralen Abschnitt (P2) sowie einen ersten Endabschnitt (P1) und einen zweiten Endabschnitt (P3) umfaßt, und entsprechend einer im allgemeinen C-förmigen Gestaltung faltbar ist, wobei der erste Endabschnitt (P1) auf den zentralen Abschnitt (P2) gefaltet wird und der zweite Endabschnitt (P3) auf den ersten Abschnitt (P1) gefaltet wird, welcher wiederum auf den zentralen Abschnitt (P2) gefaltet wird, **dadurch gekennzeichnet, daß** das Folienelement (F) in Bereichen, die den Endabschnitten (P1, P3) entsprechen, Taschenformationen umfaßt, in welchen die homologen Enden der Artikel (A) aufgenommen sind.

2. Der Packen nach Anspruch 1, **dadurch gekennzeichnet, daß** der Artikel (A) ein Hygieneartikel wie beispielsweise eine Slipeinlage ist.

3. Der Packen nach Anspruch 1 oder Anspruch 2, **dadurch gekennzeichnet, daß** der Artikel (A) aufgrund der Anordnung der homologen Enden in den Taschenformationen im wesentlichen ohne eine adhäsive Verbindung zwischen dem Artikel (A) und dem Folienelement (F) auf dem Folienelement (F) gehalten ist.

4. Der Packen nach einem der vorhergehenden Ansprüche, **dadurch gekennzeichnet, daß** der Artikel (A) einen an ihm angebrachten Schutzstreifen trägt, wobei der Schutzstreifen selektiv von dem Artikel (A) lösbar ist.

5. Der Packen nach Anspruch 4, **dadurch gekennzeichnet, daß** zwischen dem Artikel (A) und dem Schutzstreifen eine adhäsive Schicht vorgesehen ist.

6. Der Packen nach einem der vorhergehenden Ansprüche, **dadurch gekennzeichnet, daß** die Taschenformationen aus Rändern (F1) des Folienelementes (F) gebildet sind, die auf das Folienelement (F) selbst gefaltet sind.

7. Der Packen nach Anspruch 6, **dadurch gekennzeichnet, daß** die Ränder (F1) mit dem Folienelement durch Verbindungsformationen (F2), vorzugsweise in der Form von Bahnen, verbunden sind.

8. Der Packen nach einem der vorhergehenden Ansprüche, **dadurch gekennzeichnet, daß** bei dem entsprechend der C-förmigen Gestaltung der gefalteten Packen der zweite Endabschnitt (P3) mit dem ersten Endabschnitt (P1) im wesentlichen ohne Verbindungselemente zwischen dem distalen Rand des zweiten Endabschnittes (P3) und des ersten Endabschnittes (P1) verbunden ist.

9. Ein Verfahren zur Herstellung eines Packens (P), welcher einen Artikel (A) umfaßt, der auf einem Streifenelement (F) angebracht ist, wobei die Anordnung, die durch den Artikel (A) und durch das Folienelement (F) gebildet ist, welches einen zentralen Abschnitt (P2) sowie einen ersten Endabschnitt (P1) und einen zweiten Endabschnitt (P3) umfaßt, und entsprechend einer im allgemeinen C-förmigen Gestaltung faltbar ist, wobei der erste Endabschnitt (P1) auf den zentralen Abschnitt (P2) gefaltet wird, und der zweite Endabschnitt (P3) auf den ersten Abschnitt (P1) gefaltet wird, welcher auf den zentralen Abschnitt (P2) gefaltet ist, **dadurch gekennzeichnet, daß** es den Arbeitsvorgang umfaßt, daß in dem Folienelement (F) in den Gebieten, die den Endbereichen (P1, P3) entsprechen, Taschenformationen ausgebildet werden, in welchen die homologen Enden des Artikels (A) aufgenommen werden.

10. Das Verfahren nach Anspruch 9, **dadurch gekennzeichnet, daß** der Artikel (A) als ein Hygieneartikel, wie beispielsweise eine Slipeinlage gewählt wird.

11. Das Verfahren nach Anspruch 9 oder Anspruch 10, **dadurch gekennzeichnet, daß** es den Arbeitsvorgang umfaßt, daß die entsprechenden homologen Enden in den Taschenformationen im wesentlichen ohne eine adhäsive Verbindung zwischen dem Artikel (A) und dem Folienelement (F) angeordnet werden.

12. Das Verfahren nach einem der vorhergehenden Ansprüche von Anspruch 9 bis Anspruch 11, **dadurch gekennzeichnet, daß** es den Arbeitsvorgang umfaßt, daß ein Schutzstreifen auf dem Artikel (A) angeordnet wird, wobei der Schutzstreifen selektiv von dem Artikel (A) lösbar ist.

13. Das Verfahren nach Anspruch 12, **dadurch gekennzeichnet, daß** es den Arbeitsvorgang umfaßt, daß eine adhäsive Verbindungsschicht zwischen dem Artikel (A) und den Schutzstreifen angeordnet wird.

14. Das Verfahren nach einem der vorhergehenden Ansprüche von Anspruch 9 bis Anspruch 13, **dadurch gekennzeichnet, daß** es den Arbeitsvorgang umfaßt, daß die Taschenformationen ausgebildet werden, indem Ränder (F1) des Folienelementes (F) auf das Folienelement (F) selbst gefaltet werden.

15. Das Verfahren nach Anspruch 14, **dadurch gekennzeichnet, daß** es den Arbeitsvorgang umfaßt, daß die Ränder (F1) mit dem Körper des Folienelementes (F1), vorzugsweise durch Abdichten, verbunden wird.

16. Das Verfahren nach einem der vorhergehenden Ansprüche von Anspruch 9 bis Anspruch 15, **dadurch gekennzeichnet, daß** es bei einem Fluß von Artikeln (A), während diese sich bewegen, durchgeführt wird, und daß es den Arbeitsvorgang umfaßt, daß die Bewegung der Artikel (A) aktiv gesteuert wird (2), bis die Taschenformationen hergestellt sind.

17. Ein Verfahren nach Anspruch 16, **dadurch gekennzeichnet, daß** die Taschenformationen gebildet werden, indem auf die einander gegenüberliegenden Ränder (F1) des Folienelementes (F) mit Verbindungselementen (5) eingewirkt wird, welche zumindest teilweise (50) entsprechend einer im allgemeinen freitragenden Ausbildung hinsichtlich des ersten Folienelementes (F) montiert sind, und daß es den Arbeitsvorgang umfaßt, daß es Mittel (3) zur aktiven Förderung der Artikel (A) vorsieht, die sich in einer Position zwischen den Verbindungselementen (5), die zumindest teilweise (50) auf eine freitragende weise angeordnet sind, erstrecken.

## Revendications

1. Paquet comprenant un article (A) appliqué sur un élément en feuille ou membrane (F), l'ensemble formé de l'article (A) et de l'élément en membrane (F) comprenant une branche centrale (P2) ainsi qu'une première branche d'extrémité (P1) et une seconde branche d'extrémité (P3), et pouvant être plié selon une conformation générale C, ladite première branche d'extrémité (P1) étant repliée sur ladite branche centrale (P2) et ladite seconde branche d'extrémité (P3) étant repliée sur ladite première branche (P1) qui est à son tour repliée sur ladite branche centrale (P2), **caractérisé en ce que** ledit élément en membrane (F) comprend, dans des zones correspondant aux dites branches d'extrémité (P1, P3), des formations de poche dans lesquelles sont introduites les extrémités homologues dudit article (A).

2. Paquet selon la revendication 1, **caractérisé en ce que** ledit article (A) est un produit sanitaire tel qu'un protège-slip.

3. Paquet selon la revendication 1 ou 2, **caractérisé en ce que** ledit article (A) est retenu sur ledit élément en membrane (F) en conséquence de l'emplacement desdites extrémités homologues dans lesdites formations de poche, en l'absence sensible d'une connexion par adhésion entre ledit article (A) et ledit élément en membrane (F).

4. Paquet selon l'une quelconque des revendications précédentes, **caractérisé en ce que** ledit article (A) comporte une bande protectrice appliquée sur lui, ladite bande protectrice pouvant être retirée sélectivement dudit article (A).

5. Paquet selon la revendication 4, **caractérisé en ce qu'**entre ledit article (A) et ladite bande protectrice, se trouve interposée une couche adhésive.

6. Paquet selon l'une quelconque des revendications précédentes, **caractérisé en ce que** lesdites formations de poche sont faites à partir de marges (F1) dudit élément en membrane (F) qui sont repliées sur l'élément en membrane (F) lui-même.

7. Paquet selon la revendication 6, **caractérisé en ce que** lesdites marges (F1) sont connectées audit élément en membrane au moyen de formations de connexion (F2) de préférence sous forme de lignes.

8. Paquet selon l'une quelconque des revendications précédentes, **caractérisé en ce que** de par le pliage dudit paquet selon ladite conformation C, ladite seconde branche d'extrémité (P3) est connectée à ladite première branche d'extrémité (P1) en l'absence sensible d'éléments de connexion entre la marge distale de ladite seconde branche d'extrémité (P3) et ladite première branche d'extrémité (P1).

9. Procédé de fabrication d'un paquet (P) comprenant un article (A) appliqué sur un élément en bande (F), l'ensemble formé par l'article (A) et par l'élément en membrane (F) comprenant une branche centrale (P2) ainsi qu'une première branche d'extrémité (P1) et une seconde branche d'extrémité (P3), et pouvant être plié selon une conformation générale C, ladite première branche d'extrémité (P1) étant repliée sur ladite branche centrale (P2) et ladite seconde branche d'extrémité (P3) repliée sur ladite première branche (P1), étant repliée sur ladite branche centrale (P2), **caractérisé en ce qu'**il comprend l'opération consistant à effectuer dans ledit élément en membrane (F), dans des zones correspondant aux dites branches d'extrémité (P1, P3), des formations de poche dans lesquelles les extrémités homologues dudit article (A) sont introduites.

10. Procédé selon la revendication 9, **caractérisé en ce que** ledit article (A) est par choix un produit sanitaire tel qu'un protège-slip.

11. Procédé selon la revendication 9 ou 10, **caractérisé en ce qu'**il comprend l'opération consistant à placer lesdites extrémités homologues correspondantes dans lesdites formations de poche, en l'absence sensible de toute connexion adhésive entre ledit article (A) et ledit élément en membrane (F).

12. Procédé selon l'une quelconque des revendications 9 à 11, **caractérisé en ce qu'**il comprend l'opération consistant à appliquer au dit article (A) une bande protectrice, ladite bande protectrice pouvant être retirée de manière sélective dudit article (A).

13. Procédé selon la revendication 12, **caractérisé en ce qu'**il comprend l'opération consistant à interposer une couche de connexion adhésive entre ledit article (A) et ladite bande protectrice.

14. Procédé selon l'une quelconque des revendications 9 à 13, **caractérisé en ce qu'**il comprend l'opération consistant à former lesdites formations de poche en repliant (3) les marges (F1) dudit élément en membrane (F) contre ledit élément en membrane (F) lui-même.

15. Procédé selon la revendication 14, **caractérisé en ce qu'**il comprend l'opération consistant à connecter lesdites marges (F1) au corps dudit élément en membrane (F1), de préférence par scellage.

16. Procédé selon l'une quelconque des revendications 9 à 15, **caractérisé en ce qu'**il est exécuté sur un débit desdits articles (A) pendant qu'ils se déplacent et **en ce qu'**il comprend l'opération consistant à commander directement (2) le mouvement desdits articles (A) jusqu'à ce que des formations de poche soient réalisées.

17. Procédé selon la revendication 16, **caractérisé en ce que** lesdites formations de poche sont formées en agissant sur lesdites marges opposées (F1) dudit élément en membrane (F) au moyen d'éléments de connexion (5) montés, au moins en partie (50), selon une configuration générale en porte-à-faux par rapport audit premier élément en membrane (F), et **en ce qu'**il comprend l'opération consistant à fournir des moyens (3) de commande directe desdits articles (A) s'étendant dans une position se trouvant entre lesdits éléments de connexion (5) agencés au moins en partie (50) en porte-à-faux.
